# EUROPÄISCHE PATENTSCHRIFT

(11) **EP 4 041 370 B1**
(45) Veröffentlichungstag und Bekanntmachung des Hinweises auf die Patenterteilung: **13.11.2024**
(21) Anmeldenummer: 20819682.4
(22) Anmeldetag: 02.12.2020
(51) Int. Cl.: A61N 1/04, H05K 1/03, A61B 5/27, H05K 1/09, A61B 5/00, A61B 5/28, A41D 1/00

(54) **INTELLIGENTES KLEIDUNGSSTÜCK MIT KONTAKTELEKTRODE**
SMART GARMENT HAVING A CONTACT ELECTRODE
VÊTEMENT INTELLIGENT DOTÉ D'UNE ÉLECTRODE DE CONTACT

(30) Priorität: 04.12.2019 AT 510582019
(43) Veröffentlichungstag der Anmeldung: 17.08.2022
(73) Patentinhaber: Adaptive Regelsysteme Gesellschaft mbH, 5020 Salzburg (AT)
(72) Erfinder: WOHNSDORF, Silke, 5020 Salzburg (AT)
(74) Vertreter: Patentanwälte Pinter & Weiss OG
(86) Internationale Anmeldenummer: PCT/EP2020/084193
(87) Internationale Veröffentlichungsnummer: WO 2021/110711

(56) Entgegenhaltungen:
- EP-A1- 3 155 913
- WO-A1-2015/022327
- WO-A1-2019/145891
- WO-A2-2016/131936
- US-A1- 2009 227 857

## Beschreibung

Die gegenständliche Erfindung betrifft ein intelligentes Kleidungsstück mit einem Trägermaterial auf dem eine Elektrode angeordnet ist, sowie ein Verfahren zum Herstellen eines intelligenten Kleidungsstückes mit einer Elektrode und eine Elektrodenanordnung zur Ausbildung der Elektrode.

Elektroden, insbesondere Körperelektroden, werden beispielsweise benötigt um elektrische Spannungen an einem menschlichen oder auch tierischen Körper zu messen, um Muskelaktivitäten oder den Puls zu messen. Elektroden werden aber auch für andere technische Anwendungen benötigt, wie beispielsweise die Erkennung von Stromflüssen durch den menschlichen Körper um Elektrounfälle erkennen zu können. Elektroden werden aber auch benötigt um Ströme in menschliche oder tierische Körper einzubringen, wie es bei der elektrischen Muskelstimulation (EMS) oder bei Wiederbelebungsmaßnahmen nötig ist.

Elektroden werden aber auch für die Kontaktierung von anderen Gegenständen benötigt, insbesondere zur Kontaktierung von elektrisch leitenden Gegenständen, wie metallische Gegenstände. Eine denkbare Anwendung ist beispielsweise bei der technischen Überprüfung von Schweißstellen, wobei der elektrische Widerstand der Schweißstelle mittels Vierdrahtmessung ermittelt werden soll. Um eine optische Beeinträchtigung des Gegenstandes zu vermeiden, können vier Elektroden, zwei auf jeder Seite des Gegenstandes, auf den Gegenstand aufgepresst werden, ohne den Gegenstand dadurch zu beeinträchtigen, wie das beispielsweise durch harte Klemmen ansonsten der Fall wäre.

Daneben gibt es natürlich noch eine Fülle anderer Anwendungen, bei denen eine Elektrode zur elektrischen Kontaktierung eines Gegenstandes benötigt werden.

Insbesondere bei menschlichen oder tierischen Anwendungen, aber auch bei anderen Anwendungen, können Elektroden aus hautfreundlichen Materialen, wie beispielsweise Silikon oder Polytetrafluorethylen (PTFE) oder andere Kunststoffe, zur Anwendung kommen.

Bevorzugt ist ein Material für die Elektrode, das einen niedrigen Übergangswiderstand sicherstellt, um eine gute elektrische Kontaktierung zu ermöglichen. Auch unter diesem Aspekt ist elektrisch leitfähiges Silikon oder PTFE, oder allgemein ein elektrisch gut leitfähiger Kunststoff, eine gute Wahl für das Material der Elektrode.

Elektroden werden auch vermehrt in Kleidungsstücken verwendet, um sogenannte intelligente Kleidungsstücke, auch unter Smart Textilien bekannt, auszubilden. Als smarte Textilien werden Kleidungsstücke mit integrierter, mitunter auch von außen nicht sichtbarer, Elektronik oder Elektrik bezeichnet. Mit der Elektronik können nahezu beliebige Funktionen realisiert werden, wie beispielsweise die Überwachung von Vitalparametern (z.B. Herzfrequenz, Blutdruck, Körpertemperatur, Atemfrequenz, Elektrokardiogramm (EKG), usw.), Sicherheitsfunktionen (z.B. um elektrische Spannungen oder Ströme zu detektieren, usw.), wofür Elektroden zur Kontaktierung des Körpers bzw. der Haut benötigt werden.

Verschiedene Elektrodenanordnungen sind aus dem Stand der Technik bekannt: siehe zum Beispiel WO 2015/022327 A1, WO 2016/131936 A2, US 2009/227857 A1 und EP 3 155 913 A1.

Eine Elektrode an einem intelligenten Kleidungsstück muss natürlich auch elektrisch kontaktiert werden, um mit der Elektrik oder Elektronik am intelligenten Kleidungsstück elektrisch verbunden zu werden. Übliche Verbindungstechniken wie Löt- oder Krimpverbindungen sind im Bereich der Textilindustrie aber ungeeignet. Zum einen sind die Verfahren gänzlich neu für die Textilindustrie mit entsprechendes Prozess Knowhow fehlt. Zum anderen sind auf diese Weise hergestellten Verbindungen nicht gut beständig in Waschvorgängen, welchen Textilien unterworfen werden, und die Verfahren sind verhältnismäßig aufwändig in der Durchführung. Abgesehen davon ist es nachvollziehbar ungünstig, auf einem textilen Material eine Lötstelle auszubilden, da dadurch das textile Material leicht beschädigt oder sichtbar beeinträchtigt werden kann. Auch Krimpverbindungen sind aufgrund der Größe solcher Verbindungen auf intelligenten Kleidungsstücken eher ungünstig, sowohl hinsichtlich der Optik, als auch des Tragekomforts. Auch die Verwendung von elektrisch leitfähigen flüssigen Klebstoffen oder Massen wäre zur Herstellung elektrischer Verbindungen auf intelligenten Kleidungsstücken grundsätzlich möglich. Beispielsweise könnte Silikon in flüssiger Form auf einem Textil aufgebracht werden. Allerdings ist es höchst aufwändig, in Nähereien mit flüssigen Klebstoffen oder flüssigen Massen zu arbeiten. Das Risiko der Verschmutzung der Textilien mit Klebstoff oder anderen flüssigen Massen ist, vor allem auch durch unsachgemäße Handhabung, hoch. Abgesehen davon sind die mit solchen Verfahren hergestellten Verbindungen für den textilen Alltag auch nicht prozesssicher und haltbar genug.

Zudem kommt noch das Problem hinzu, dass Materialien, die sich gut für Elektroden eignen, wie Silikon, PTFE oder andere Kunststoffe, auf vielen Materialen, die für eine Verbindung der Elektrode mit dem intelligenten Kleidungsstück verwendet werden könnten, schlecht oder gar nicht haften.

Elektroden auf einem Textil mit einem für die Textilindustrie geeigneten Verfahren anzubringen und damit gleichzeitig eine elektrische Verbindung herzustellen ist daher eine diffizile Aufgabe.

Ein Ziel der gegenständlichen Erfindung ist es daher, die Anordnung einer Elektrode auf einem intelligenten Kleidungstück unter Herstellung einer elektrischen Verbindung auf einfache und prozesssichere Weise zu ermöglichen.

Dieses Ziel kann mit einer Elektrodenanordnung gemäß Anspruch 1 erreicht werden, bei der ein Elektrodenträger in Form eines flächigen Materialstücks aus einem textilen oder nicht textilen Material vorgesehen ist, wobei der Elektrodenträgers auf einer Seite mit einer elektrisch leitfähigen Elektrode verbunden ist und an der gegenüberliegenden Seite des Elektrodenträgers ein elektrisch leitfähiger Thermoklebstoff anliegt, wobei die Elektrode und der Thermoklebstoff für eine elektrische Verbindung elektrisch leitend verbunden sind. Für eine elektrische Verbindung sind die Elektrode und der Thermoklebstoff, vorzugsweise über den Elektrodenträger oder durch direkte Kontaktierung des Elektrodenmaterials mit dem Thermoklebstoff oder durch Vorsehen von elektrischen Verbindungen, elektrisch leitend verbunden. Eine solche Elektrodenanordnung kann in der benötigten Form und Größe vorgefertigt werden kann einfach und prozesssicher auf einem intelligenten Kleidungstück appliziert werden. Die Ausführung der Elektrodenanordnung ermöglicht es auch an sich hinsichtlich der Haftung nicht kompatible Materialien zu verwenden. Beispielsweise kann auf diese Weise eine besonders geeignete Silikonelektrode mit einem Thermoklebstoff mit dem intelligenten Kleidungsstück verbunden werden, obwohl Silikon nicht geeignet wäre, mit einem Thermoklebstoff geklebt zu werden. Damit können sowohl die Anforderungen an die Beständigkeit und Prozesssicherheit der Verbindung, als auch die Anforderungen an die gute elektrische Kontaktierung sichergestellt werden

Elektroden aus einer solchen Elektrodenanordnung können sehr kostengünstig in großen Flächen zu vielen Einheiten hergestellt werden, wobei besonders vorteilhaft die die Elektrode ausbildenden Kunststoffflächen mittels einem Druckverfahren oder Extrusion aufgebracht werden können. Der Thermoklebstoff kann dabei vollflächig aufgebracht werden. Danach können Teile in der benötigten Form und Größe ausgestanzt werden und als textile Bauteile verwendet werden. Das Aufbringen solcher textilen Bauteile auf einem intelligenten Kleidungsstück ist zudem äußert einfach unter Druck und Temperatur, beispielsweise mit einer Thermotransferpresse, möglich.

In einer bevorzugten Ausführung ist der Elektrodenträger der Elektrodenanordnung aus einem textilen Material gefertigt und das textile Material des Elektrodenträgers wird zumindest teilweise vom Material der Elektrode, vorzugsweise ein elektrisch leitfähiger Kunststoff, durchdrungen. Damit kann die elektrische Verbindung auf einfache Weise hergestellt oder die Herstellung einer elektrischen Verbindung zumindest unterstützt werden. Die Herstellung einer elektrischen Verbindung ist insbesondere auf einfache Wese möglich, wenn das textile Material des Elektrodenträgers auch zumindest teilweise vom Thermoklebstoff durchdrungen ist.

Wenn der Elektrodenträger elektrisch leitfähig ausgeführt ist, kann die Qualität und Beständigkeit der elektrischen Verbindung verbessert werden.

Die gegenständliche Erfindung wird nachfolgend unter Bezugnahme auf die Figuren näher erläutert, die beispielhaft, schematisch und nicht einschränkend vorteilhafte Ausgestaltungen der Erfindung zeigen. Dabei zeigt
Fig.1 eine Elektrodenanordnung,
Fig.2 eine Elektrodenbahn zum Herstellen einer Elektrodenanordnung,
Fig.3 und 4 die Verwendung der Elektrodenanordnung an einen Trägermaterial und
Fig.5 ein intelligentes Kleidungsstück mit einer Elektrode aus einer Elektrodenanordnung.

Textilien (textile Materialen) im Sinne der Erfindung umfassen textile Rohstoffe oder nichttextile Rohstoffe die durch verschiedene Verfahren zu flächenförmigen oder räumlichen textilen Anordnungen verarbeitet wurden. Textilien aus textilen Rohstoffen können auch nichttextile Rohstoffe enthalten und umgekehrt. Textile Rohstoffe können Naturfasern (pflanzlichen oder tierischen Ursprungs) oder Kunstfasern (aus Kunststoffen) umfassen, wie beispielsweise Baumwolle, Wolle, Polyester oder Polyamid. Nichttextile Rohstoffe können z.B. auch Kunststoffe, Leder, Federn, Schuppen oder Metalle sein. Typische flächenförmige Anordnungen sind Gewebe, Maschenware, Gewirke, Gestricke, Geflechte, Nähgewirke, Vliesstoffe oder Filze, die meistens in Form von textilen Stoffbahnen vorliegen. Räumliche Anordnungen können beispielsweise röhrenförmige Gebilde sein, ebenfalls wieder in Form von Gewebe, Maschenware, Gewirke, Gestricke, Geflechte, Nähgewirke, Vliesstoffe oder Filze. Ein textiles Material kann ausreichend flexibel sein, um zu einem Kleidungsstück verarbeitet zu werden oder um in einem Kleidungstück verwendet werden zu können. Ein nicht textiles Material kann ein flächenförmiges Stück aus einem nichttextilen Rohstoff sein, das auch eine gewissen Flexibilität aufweisen kann.

Eine erfindungsgemäße Elektrodenanordnung 1 besteht aus einem textilen oder auch nicht textilen Elektrodenträger (Flicken oder ein Materialstückchen) 2, auf dem auf einer Seite eine elektrisch leitfähige Elektrode 4 aufgebracht ist. Der Elektrodenträger 2 ist ein ausreichend großes und flächiges Materialstück aus einem textilen oder nicht textilen Material, um die benötigte Elektrode 4 darauf anbringen zu können. Der Elektrodenträger 2 kann aus einem textilen Material, wie ein Metallgewebe, -gewirk, -gestrick oder Ähnliches, gefertigt sein, oder auch aus einem nicht textilen Material wie ein Stück Leder, Kunststoff oder Metall. Vorteilhaft ist der Elektrodenträger 2 gleich groß oder größer als die Elektrode 4.

Als Material der Elektrode 4 kommt elektrisch leitfähiges Silikon, elektrisch leitfähiges PTFE oder ein anderer elektrisch leitfähiger Kunststoff oder auch ein anderes elektrisch leitfähiges Material, wie z.B. Metall, in Frage. Ein Kunststoff kann beispielsweise durch Zugabe von elektrisch leitenden Füllstoffen, wie beispielsweise aus Aluminium, Kupfer, Graphit oder Ruß, elektrisch leitfähig gemacht werden. Ebenso sind elektrisch selbstleitende Polymere bekannt, die ebenfalls verwendet werden können. Auch ein Materialstück mit einer elektrisch leitfähigen Beschichtung kommt als Elektrode 4 in Frage.

Die Elektrode 4 kann entweder flüssig, beispielsweise mit einem Druckverfahren, auf den Elektrodenträger 2 aufgebracht werden, auf den Elektrodenträger 2 extrudiert (im Falle eines Kunststoffes) werden oder mit einem geeigneten Kleber aufgeklebt werden. Im Falle eines Klebers kommt natürlich ein Klebstoff zur Anwendung, der eine ausreichende Haftung zwischen der Elektrode 4 und dem Elektrodenträger 2 gewährleistet. Um eine elektrische Kontaktierung sicherzustellen, ist der Klebstoff ebenfalls elektrisch leitfähig oder es sind elektrische Verbindungen durch die Klebstoffschicht vorzusehen. Dabei kann das Elektrodenmaterial oder der Kleber vor dem Aushärten auch das textile Material des Elektrodenträgers 2 zumindest teilweise durchdringen, was die elektrische Kontaktierung unterstützen kann.

An der gegenüberliegenden Seite der Elektrodenanordnung 1 ist ein elektrisch leitender Thermoklebstoff 3 vorgesehen.

Ein Thermoklebstoff 3 ist ein Klebstoff, meist in Form eines Thermoplastes, der bei normalen Einsatztemperaturen eines intelligenten Kleidungsstückes, typischerweise zwischen -20°C und 60°C, einen festen oder auch elastisch-festen Zustand besitzt und üblicherweise nicht klebend ist (also keine Hafteigenschaften aufweist). Ab einer bestimmten Schmelztemperatur (Reaktionstemperatur), typischerweise zwischen 125°C und 250°C (also weit oberhalb der Einsatztemperatur), erweicht der Thermoklebstoff bis zur Fließbarkeit und entwickelt zusätzlich Hafteigenschaften (wird somit klebend). Bei Abkühlung härtet der Thermoklebstoff wieder aus und verfestigt sich wieder, wobei der Thermoklebstoff seine Klebrigkeit verliert. Hafteigenschaften können sich dabei auch schon unterhalb der Schmelztemperatur beginnen auszubilden. Zwei Haftpartner, die durch den Thermoklebstoff miteinander verbunden werden sollen, werden bei aufgeschmolzenem Thermoklebstoff durch Druck aneinandergepresst, sodass sich der Thermoklebstoff mit den beiden Haftpartnern verbindet. Nach dem Aushärten des Thermoklebstoffes bildet sich eine sichere und dauerhafte, unter Umständen auch eine elastische, Verbindung zwischen den beiden Haftpartnern aus.

Der Thermoklebstoff 3 kann auf dem Elektrodenträger 2 aufgetragen sein, beispielsweise in Pastenform aufgetragen, aufgeklebt oder aufextrudiert, oder kann in Form eines Netzes, Gewebes oder einer Folie, oder Ähnliches, vorliegen und lose an Elektrodenträger 2 angelegt werden. Zur Herstellung einer elektrischen Verbindung muss auch der Thermoklebstoff 3 elektrisch leitend sein.

Um einen solchen Thermoklebstoff 3 elektrisch leitend zu machen kann ein elektrisch leitendes Material, beispielsweise Graphitpulver oder ähnliche leitfähige Substanzen oder Füllstoffe, zum Thermoklebstoff 3 beigemengt werden. Beispielsweise kann ein kommerziell erhältliches Thermotransfermaterial als Granulat für einen Extruder vorliegen. Beim Extrudieren des Thermotransfermaterials zu einer Folie oder einer Faser (z.B. als textiler Rohstoff zur Herstellung eines textilen Materials) oder beim Aufextrudieren auf den Elektrodenträger 2 kann dann das elektrisch leitende Material, das pulver-, pasten- oder granulatförmig vorliegen kann, beigemischt werden, um den extrudierten Thermoklebstoff elektrische leitend zu machen. Aus einer derart hergestellten Faser kann dann ein textiles Material, z.B. ein Netz oder ein Gewebe, aus Thermoklebstoff hergestellt werden. Ein pulverförmiges Thermotransfermaterial kann z.B. mit dem elektrisch leitenden Material gemischt werden, um daraus eine Paste aus Thermoklebstoff herzustellen. Ein pastenförmiges Thermotransfermaterial kann ebenfalls mit einem pulverförmigen oder pastenförmigen elektrisch leitenden Material gemischt werden, um das Thermotransfermaterial elektrisch leitend zu machen. Beispielhafte Marken und Hersteller von Thermotransfermaterialen sind unter anderem PLATAMID (von Arkema), DYNACOLL S (von Evonik) oder TUBILUX TD 90 NF (von CHT). Bei geeigneter Materialwahl für das Thermotransfermaterial und das elektrisch leitende Material entstehen mit einem daraus hergestellten Thermoklebstoff elektrische Verbindungen mit Widerständen vom weniger als 10 Ohm, vorzugsweise im Milliohmbereich. Je nach Anwendung können aber auch Widerstände im Kilo- oder Megaohmbereich ausreichend sein.

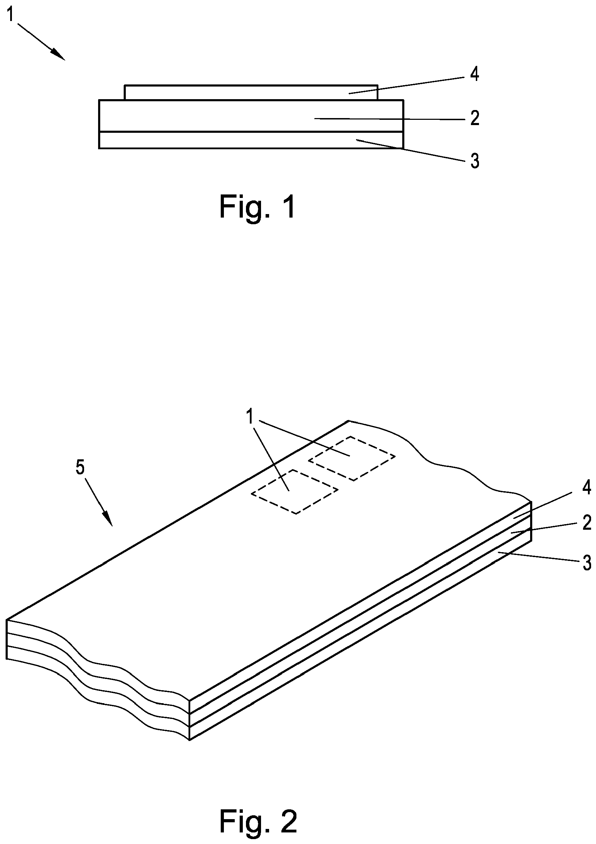

In einer vorteilhaften Ausgestaltung werden ganze Elektrodenbahnen 5 in großer Länge und Breite hergestellt (wie in Fig.2 dargestellt), auf denen auf einer Seite einer Bahn des Elektrodenträgers 2 das Elektrodenmaterial, vorzugsweise flächig, aufgebracht wird, beispielsweise durch Extrudieren, Drucken, Kleben usw., und auf der anderen Seite der Thermoklebstoff 3 aufgebracht wird, beispielsweise durch Extrudieren, in Form einer Paste usw. Bei Verwendung eines Druckverfahrens zum Aufbringen des Elektrodenmaterials könnte anstelle eines flächigen Auftrags die Elektrode auch in der benötigten Größe und Form gedruckt werden, um Material einzusparen. Aus der Elektrodenbahn 5 können dann die Elektrodenanordnungen 1 in der benötigten Größe und Form ausgestanzt oder ausgeschnitten werden und weiterverarbeitet werden.

Die Elektrodenbahn 5 könnte auch ohne Thermoklebstoff 3 hergestellt werden. Dann kann der Thermoklebstoff 3 als separater Klebstoffpatch (in Form eines textilen oder nicht-textilen Materials, wie einer Folie, Netz, Gewebe usw.) verwendet werden. Alternativ könnte der Thermoklebstoff 3 auch vor der Anwendung in Form einer Paste auf die Elektrodenanordnung 1 oder auf das Trägermaterial 6, auf dem die Elektrode befestigt werden soll, aufgetragen werden.

In einer bevorzugten Ausgestaltung der Elektrodenanordnung 1 wird ein Elektrodenträger 2 aus einem textilen Material verwendet, auf dem die Elektrode 4 in flüssiger Form oder durch Extrusion aufgebracht wurde, sodass das elektrisch leitfähige Elektrodenmaterial das textile Material des Elektrodenträgers 2 zumindest teilweise durchdringt. Auf der anderen Seite ist der elektrisch leitfähige Thermoklebstoff 3 durch Extrusion aufgetragen. Idealerweise kann der Thermoklebstoff 3 dabei bereits das textile Material des Elektrodenträgers 2 zumindest teilweise durchdringen. Auf diese Weise kann bereits bei der Herstellung der Elektrodenanordnung 1 eine elektrische Verbindung zwischen Elektrode 4 und Thermoklebstoff 3 hergestellt werden. Es kann aber auch vorgesehen sein, dass der elektrisch leitfähige Thermoklebstoff 3 erst bei der Applikation der Elektrodenanordnung 1, also beim Verpressen unter Druck und Temperatur, so weit aufschmilzt, dass der Thermoklebstoff 3 das textile Material des Elektrodenträgers 2 zur Herstellung der elektrischen Verbindung zumindest teilweise durchdringt oder die Durchdringung und damit die elektrische Kontaktierung verbessert wird.

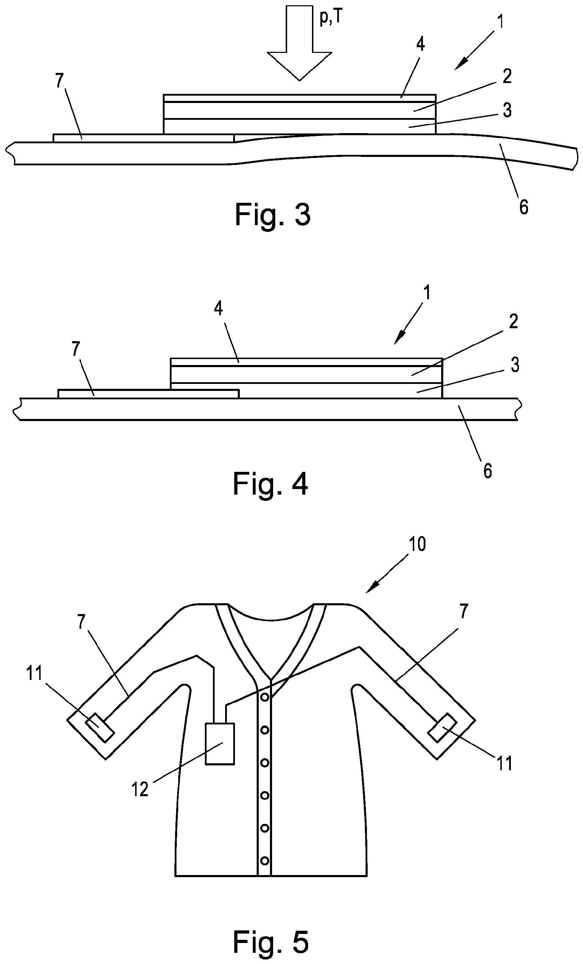

In Fig.3 umfasst die Elektrodenanordnung 1 bereits den Thermoklebstoff 3, beispielsweise weil dieser mit der Elektrodenanordnung 1 oder nachträglich auf die Elektrodenanordnung 1 aufgetragen wurde. Die Elektrodenanordnung 1 wird auf ein Trägermaterial 6, beispielsweise ein intelligentes Kleidungsstück, aufgelegt. Damit soll eine elektrische Verbindung zwischen der Elektrode 4 der Elektrodenanordnung 1 und dem Trägermaterial 6 oder einem elektrischen Leiter 7 auf oder im Trägermaterial 6 hergestellt werden. Zusätzlich kann daher auch zumindest ein elektrischer Leiter 7, beispielsweise ein Draht, zwischen Elektrodenanordnung 1 und Trägermaterial 6 angeordnet werden (wie in Fig.3). Der elektrische Leiter 7 kann auf dem Trägermaterial 6 aufliegen oder auch im Trägermaterial 6 integriert sein. Der elektrische Leiter 7 ist am Ende, das zwischen Elektrodenanordnung 1 und Trägermaterial 6 liegt, natürlich abisoliert, um eine gute elektrische Kontaktierung herzustellen. Das Trägermaterial 6 kann auch selbst lokal elektrisch leitfähig sein, beispielsweise indem in das Trägermaterial 6 elektrisch leitende Fasern, beispielsweise aus Metall, eingearbeitet sind, was auch einen Leiter 7 ersetzen kann. Die Elektrodenanordnung 1 wird daraufhin unter Druck p und Temperatur T (oberhalb der Schmelztemperatur des verwendeten Thermotransfermaterials) gegen das Trägermaterial 6 gepresst (angedeutet durch den Pfeil), beispielsweise mit einer Thermo-Transfer-Presse. Alternativ und gleichwertig könnte anstelle einer Elektrodenanordnung 1 mit Thermoklebstoff 3 auch ein separater Klebstoffpatch (als Netz, Gewebe, Folie, etc.) aus Thermoklebstoff 3 zwischen der Elektrodenanordnung 1 und dem Trägermaterial 6 eingelegt werden. Auch in diesem Fall schmilzt der Thermoklebstoff 3 beim Verpressen unter Druck p und Temperatur T auf und verbindet sich auch mit dem Trägermaterial 6 und mit dem Elektrodenträger 2 der Elektrodenanordnung 1 (wie in Fig.4 dargestellt) und umgibt einen allenfalls vorhandenen Leiter 7 zumindest teilweise. Nach dem Aushärten des Thermoklebstoffes 3 entsteht eine sichere, dauerhaft elektrische Verbindung zwischen der Elektrode 4 und dem Leiter 7 und/oder dem Trägermaterial 6. Gleichzeitig kann dadurch der zumindest eine Leiter 7 sicher und dauerhaft mechanisch auf dem Trägermaterial 6 fixiert werden.

Die elektrische Verbindung durch den Elektrodenträger 2 kann erzielt werden, indem der Elektrodenträger 2 selbst elektrisch leitend ist, beispielsweise durch eine geeignete Materialauswahl, oder indem in den Elektrodenträger 2 elektrische leitende Elemente eingearbeitet sind, die die elektrische Verbindung sicherstellen, beispielsweise eingearbeitete Metallfäden in einen textilen Elektrodenträger 2. Der Elektrodenträger 2 kann beispielsweise als Metalldrahtgewebe oder Metalldrahtgestrick ausgeführt sein. Der Elektrodenträger 2 kann aber auch mit einer elektrisch leitfähigen Beschichtung ausgeführt sein. Wenn der Elektrodenträger 2 selbst elektrisch leitend ist, dann ist die elektrische Verbindung besonders gut.

Die elektrische Verbindung durch den Elektrodenträger 2 kann aber vorzugsweise auch dadurch erzielt werden, dass entweder das elektrisch leitfähige Elektrodenmaterial und/oder der elektrisch leitfähige Thermoklebstoff 3 das Material des Elektrodenträgers 2 durchdringt, beispielsweise durch den Auftrag oder erst bei Applikation der Elektrodenanordnung 1 durch das Verpressen unter Druck p und Temperatur T, sodass sich das Elektrodenmaterial und der Thermoklebstoff 3 kontaktieren. Daher ist der Elektrodenträger 2 vorteilhafterweise aus einem textilen Material gefertigt, das leicht von einem flüssigen oder aufgeschmolzenen Elektrodenmaterial und Thermoklebstoff 3 durchdringbar ist.

Im intelligenten Kleidungsstück 1 können elektrische und/oder elektronische Bauteile, Komponenten oder Schaltungen integriert sein, die auf diese Weise mit der Elektrode 4 elektrische verbunden werden können.

Die Verwendung eines Elektrodenträger 2 ist insbesondere dann vorteilhaft, wenn der Thermoklebstoff 3 nicht am Material der Elektrode 4 haften würde, wie das beispielsweise bei einer Elektrode 4 aus Silikon oder PTFE der Fall wäre. Nachdem ein solches Elektrodenmaterial aber sicher und zuverlässig als Elektrode 4 auf einem Elektrodenträger 2 beschichtet oder aufgebracht werden kann und auch der Thermoklebstoff 3 sicher mit dem Elektrodenträger 2 verbunden werden kann, kann durch die erfindungsgemäße Elektrodenanordnung 1 die Elektrode 4 einfach und prozesssicher am Trägermaterial 6 angebracht werden.

Um die Applikation der Elektrodenanordnung 1 auf dem Trägermaterial 6 des intelligenten Kleidungsstückes zu erleichtern, kann auf dem Trägermaterial 6 zugewandten Seite der Elektrodenanordnung 1 auch ein bei Raumtemperatur klebender Kleber vorgesehen sein, um die Elektrodenanordnung 1 für das Thermotransferverfahren provisorisch zu fixieren. Ein solcher Kleber ist vorzugsweise nur leicht haftend und auch nach mehrmaligem Anlegen der Elektrodenanordnung 1 auf dem Trägermaterial 6 noch immer haftend, um die Elektrodenanordnung 1 einfach am Trägermaterial 6 ausrichten zu können. Ein solcher Kleber ist vorzugsweise nicht vollflächig an der Elektrodenanordnung 1 vorgesehen, sondern nur partiell und in sehr geringer Menge bzw. Grammatur. Ferner hat ein solcher Kleber vorzugsweise eine Schmelztemperatur, die unterhalb der Schmelztemperatur des Thermoklebstoffes 3 liegt, um das Thermotransferverfahren und die Herstellung der elektrischen Verbindung nicht zu beeinflussen.

Fig.5 zeigt ein Beispiel eines intelligenten Kleidungsstücks 10 (Smart Textile) mit Kontaktelektroden 11 an den Ärmelbünden, um die Haut der tragenden Person oder einen anderen Gegenstand zu kontaktieren um ein elektrisches Potential abzugreifen. Die Kontaktelektroden 11 sind in Form von Elektrodenanordnungen 1 mit einem Thermotransferverfahren am intelligenten Kleidungsstück 10 aufgebracht worden. Die beiden Kontaktelektroden 11 sind beispielsweise über Leiter 7 mit einer Auswerteeinheit 12 (z.B. Mikroprozessor mit Software oder eine elektrische Schaltung) verbunden. Die Leiter 7 können am Trägermaterial 6 des intelligenten Kleidungsstücks 10 angeordnet sein oder in das Trägermaterial 6 integriert sein. In diesem Beispiel kann eine elektrische Spannung zwischen den beiden Kontaktelektroden 11 in der Auswerteeinheit 12 ausgewertet werden. Die Herstellung des intelligenten Kleidungsstückes 10 kann mithilfe der Verwendung der Elektrodenanordnungen 1 sehr einfach und schnell erfolgen.

## Patentansprüche

1. Elektrodenanordnung, wobei ein Elektrodenträger (2) in Form eines flächigen Materialstücks aus einem textilen oder nicht textilen Material vorgesehen ist, wobei der Elektrodenträger (2) auf einer Seite mit einer elektrisch leitfähigen Elektrode (4) verbunden ist und **dadurch gekennzeichnet dass** an der gegenüberliegenden Seite des Elektrodenträgers (2) ein elektrisch leitfähiger Thermoklebstoff (3) anliegt **und dass** die Elektrode (4) und der Thermoklebstoff (3) für eine elektrische Verbindung elektrisch leitend verbunden sind.

2. Elektrodenanordnung nach Anspruch 1, **dadurch gekennzeichnet, dass** der Elektrodenträger (2) aus einem textilen Material gefertigt ist und das textile Material des Elektrodenträgers (2) zumindest teilweise vom Material der Elektrode, vorzugsweise ein elektrisch leitfähiger Kunststoff, durchdrungen ist.

3. Elektrodenanordnung nach Anspruch 1 oder 2, **dadurch gekennzeichnet, dass** der Elektrodenträger (2) aus einem textilen Material gefertigt ist und das textile Material des Elektrodenträgers (2) zumindest teilweise vom Thermoklebstoff (3) durchdrungen ist.

4. Elektrodenanordnung nach einem der Ansprüche 1 bis 3, **dadurch gekennzeichnet, dass** der Elektrodenträger (2) mit dem Thermoklebstoff (3) beschichtet ist.

5. Elektrodenanordnung nach Anspruch 1 oder 2, **dadurch gekennzeichnet, dass** der Thermoklebstoff (3) lose am Elektrodenträger (2) anliegend angeordnet ist.

6. Elektrodenanordnung nach einem der Ansprüche 1 bis 5, **dadurch gekennzeichnet, dass** der Elektrodenträger (2) elektrisch leitfähig ausgeführt ist.

7. Elektrodenanordnung nach Anspruch 6, **dadurch gekennzeichnet, dass** der Elektrodenträger (2) mit einer elektrisch leitfähigen Beschichtung ausgeführt ist.

8. Elektrodenanordnung nach Anspruch 6, **dadurch gekennzeichnet, dass** in den Elektrodenträger (2) elektrisch leitende Elemente eingearbeitet sind, die die elektrische Verbindung herstellen.

9. Intelligentes Kleidungsstück mit einem Trägermaterial (6) auf dem eine Kontaktelektrode (11) angeordnet ist, wobei die Kontaktelektrode (11) mit einer Elektrodenanordnung (1) nach einem der Ansprüche 1 bis 8 hergestellt ist und die Elektrodenanordnung (1) über den Thermoklebstoff (3) mit dem intelligenten Kleidungsstück (10) oder einem Teil davon elektrisch verbunden ist.

10. Intelligentes Kleidungsstück nach Anspruch 9, **dadurch gekennzeichnet, dass** das intelligente Kleidungsstück (10) mit einem elektrischen Leiter (7) ausgeführt ist, der zumindest teilweise vom Thermoklebstoff (3) der Elektrodenanordnung (1) umgeben ist.

11. Intelligentes Kleidungsstück nach Anspruch 9 oder 10, **dadurch gekennzeichnet, dass** das Trägermaterial (6) des intelligenten Kleidungsstücks (10) zumindest teilweise elektrisch leitfähig ist und dass der Thermoklebstoff (3) der Elektrodenanordnung (1) das elektrisch leitfähige Trägermaterial (6) zumindest teilweise kontaktiert.

12. Verfahren zum Herstellen eines intelligenten Kleidungsstückes (10) mit einer Kontaktelektrode (11), wobei auf einem Trägermaterial (6) des intelligenten Kleidungsstückes (10) zur Ausbildung der Kontaktelektrode (11) eine Elektrodenanordnung (1) nach einem der Ansprüche 1 bis 8 aufgelegt wird, wobei der Thermoklebstoff (3) dem Trägermaterial (6) zugewandt aufgelegt wird, und der Thermoklebstoff (3) der Elektrodenanordnung (1) unter Druck und Temperatur aufgeschmolzen wird, sodass die Elektrodenanordnung (1) nach dem Aushärten des Thermoklebstoffes (3) mit dem Trägermaterial (6) elektrisch verbunden wird.

## Claims

1. Electrode arrangement, wherein an electrode carrier (2) is provided in the form of a flat material piece consisting of a textile or non-textile material, the electrode carrier (2) being connected on one side to an electrically conductive electrode (4) and **characterized in that** an electrically conductive thermoadhesive (3) being in contact with the opposite side of the electrode carrier (2), **and in that** the electrode (4) and the thermoadhesive (3) are electrically conductively connected for an electrical connection.

2. Electrode arrangement according to claim 1, **characterized in that** the electrode carrier (2) is made of a textile material and the textile material of the electrode carrier (2) is at least partially penetrated by the material of the electrode, preferably an electrically conductive plastics material.

3. Electrode arrangement according to either claim 1 or 2, **characterized in that** the electrode carrier (2) is made of a textile material and the textile material of the electrode carrier (2) is at least partially penetrated by the thermoadhesive (3).

4. Electrode arrangement according to any of claims 1 to 3, **characterized in that** the electrode carrier (2) is coated with the thermoadhesive (3).

5. Electrode arrangement according to either claim 1 or 2, **characterized in that** the thermoadhesive (3) is loosely in contact with the electrode carrier (2).

6. Electrode arrangement according to any of claims 1 to 5, **characterized in that** the electrode carrier (2) is electrically conductive.

7. Electrode arrangement according to claim 6, **characterized in that** the electrode carrier (2) has an electrically conductive coating.

8. Electrode arrangement according to claim 6, **characterized in that** electrically conductive elements are incorporated into the electrode carrier (2), which produce the electrical connection.

9. Smart clothing comprising a carrier material (6) on which a contact electrode (11) is arranged, wherein the contact electrode (11) is produced having an electrode arrangement (1) according to any of the claims 1 to 8 and the electrode arrangement (1) is electrically connected to the smart clothing (10) or a part thereof via the thermoadhesive (3).

10. Smart clothing according to claim 9, **characterized in that** the smart clothing (10) is designed having an electrical conductor (7) which is at least partially surrounded by the thermoadhesive (3) of the electrode arrangement (1).

11. Smart clothing according to either claim 9 or 10, **characterized in that** the carrier material (6) of the smart clothing (10) is at least partially electrically conductive and that the thermoadhesive (3) of the electrode arrangement (1) at least partially contacts the electrically conductive carrier material (6).

12. Method for producing a smart clothing (10) comprising a contact electrode (11), wherein an electrode arrangement (1) according to any of claims 1 to 8 is placed on a carrier material (6) of the smart clothing (10) in order to form the contact electrode (11), wherein the thermoadhesive (3) is placed so as to face the carrier material (6), and the thermoadhesive (3) of the electrode arrangement (1) is melted under pressure and temperature, such that, after the thermoadhesive (3) has hardened, the electrode arrangement (1) is electrically connected to the carrier material (6).

## Revendications

1. Système à électrodes, dans lequel un support pour électrodes (2) est prévu sous la forme d'une pièce de matériau plate constituée d'un matériau textile ou non textile, dans lequel le support pour électrodes (2) est connecté, sur un côté, à une électrode (4) électriquement conductrice et **caractérisé en ce qu'**une colle thermique (3) électriquement conductrice est appliquée sur le côté opposé du support pour électrodes (2) **et en ce que** l'électrode (4) et la colle thermique (3) sont connectées de manière électriquement conductrice pour une connexion électrique.

2. Système à électrodes selon la revendication 1, **caractérisé en ce que** le support pour électrodes (2) est fabriqué en un matériau textile et le matériau textile du support pour électrodes (2) est au moins partiellement traversé par le matériau de l'électrode, de préférence une matière plastique électriquement conductrice.

3. Système à électrodes selon la revendication 1 ou 2,
**caractérisé en ce que** le support pour électrodes (2) est fabriqué en un matériau textile et le matériau textile du support pour électrodes (2) est au moins partiellement traversé par la colle thermique (3).

4. Système à électrodes selon l'une des revendications 1 à 3,
**caractérisé en ce que** le support pour électrodes (2) est revêtu de la colle thermique (3).

5. Système à électrodes selon la revendication 1 ou 2, **caractérisé en ce que** la colle thermique (3) est légèrement appliquée sur le support pour électrodes (2).

6. Système à électrodes selon l'une des revendications 1 à 5,
**caractérisé en ce que** le support pour électrodes (2) est réalisé de manière électriquement conductrice.

7. Système à électrodes selon la revendication 6, **caractérisé en ce que** le support pour électrodes (2) est réalisé avec un revêtement électriquement conducteur.

8. Système à électrodes selon la revendication 6, **caractérisé en ce que** des éléments électriquement conducteurs sont incorporés dans le support pour électrodes (2), lesquels produisent la connexion électrique.

9. Vêtement intelligent comportant un matériau de support (6) sur lequel est disposée une électrode de contact (11), dans lequel l'électrode de contact (11) est produite avec un système à électrodes (1) selon l'une des revendications 1 à 8 et le système à électrodes (1) est connecté électriquement au vêtement intelligent (10) ou à une partie de celui-ci par l'intermédiaire de la colle thermique (3).

10. Vêtement intelligent selon la revendication 9, **caractérisé en ce que** le vêtement intelligent (10) est réalisé avec un conducteur électrique (7) qui est au moins partiellement entouré par la colle thermique (3) du système à électrodes (1).

11. Vêtement intelligent selon la revendication 9 ou 10,
**caractérisé en ce que** le matériau de support (6) du vêtement intelligent (10) est au moins partiellement électriquement conducteur **et en ce que** la colle thermique (3) du système à électrodes (1) est au moins partiellement en contact avec le matériau de support (6) électriquement conducteur.

12. Procédé pour la production d'un vêtement intelligent (10) comportant une électrode de contact (11), dans lequel un système à électrodes (1) selon l'une des revendications 1 à 8 est appliqué sur un matériau de support (6) du vêtement intelligent (10) pour la formation de l'électrode de contact (11), dans lequel la colle thermique (3) est appliquée en face du matériau de support (6), et la colle thermique (3) du système à électrodes (1) est fondue sous pression et température de sorte que le système à électrodes (1) est connecté électriquement au matériau de support (6) après le durcissement de la colle thermique (3).
